# EUROPEAN PATENT SPECIFICATION

(11) **EP 0 745 407 B1**
(45) Date of publication and mention of the grant of the patent: **19.11.2003**
(21) Application number: 96102198.7
(22) Date of filing: 14.02.1996
(51) Int. Cl.: A61M 25/01, A61M 25/00

(54) **Guiding introducer for use in the treatment of atrial flutter**
Einführungshilfe zur Verwendung bei Vorhofflattern
Système d'introduction utilisable pour le traitement des flottements auriculaires

(30) Priority: 01.05.1995 US 431787
(43) Date of publication of application: 04.12.1996
(73) Proprietor: DAIG CORPORATION, Minnetonka, Minnesota 55345-2126 (US)
(72) Inventor: Swartz, John F., Tulsa, Oklahoma 74136 (US); Ockuly, John D., Minnetonka, MN 55345 (US); Hassett, James A., Bloomington, MN 55438 (US)
(74) Representative: Splanemann Reitzner Baronetzky Westendorp Patentanwälte

(56) References cited:
- EP-A- 0 531 946
- WO-A-92/12754
- US-A- 4 508 535
- US-A- 5 215 540
- US-A- 5 348 545

## Description

### 1. Field of Invention

This invention relates to introducers. More particularly, this invention relates to a guiding introducer for use within the right atrium of the human heart for the treatment of atrial flutter.

### 2. Prior Art

Introducers and catheters have been in use for medical procedures for many years. For example, one use has been to convey an electrical stimulus to a selected location within the human body. Another use is to monitor and make measurements for diagnostic tests within the human body. Catheters may be used by a physician to examine, diagnose and treat while positioned at a specific location within the body which is otherwise inaccessible without more invasive procedures. In use, catheters may be inserted into a major vein or artery which is near the body surface. These catheters are then guided to the specific location for examination, diagnosis or treatment by manipulating the catheter through the artery or vein of the human body.

Catheters have become increasingly useful in remote and difficult to reach locations within the body. However, the utilization of these catheters is frequently limited because of the need f or the precise placement of the tip of the catheter at a specific location within the body.

Control of the movement of catheters to achieve such precise placement is difficult because of the inherent structure of a catheter. The body of a conventional catheter is long and tubular. To provide sufficient control of the movement of the catheter, it is necessary that its structure be somewhat rigid. However, the catheter must not be so rigid as to prevent the bending or curving necessary for movement through the vein, artery or other body part to arrive at the specified location. Further, the catheter must not be so rigid as to cause damage to the artery or vein while it is being moved within the body.

While it is important that the catheter not be so rigid as to cause injury, it is also important that there be sufficient rigidity in the catheter to accommodate torque control, i.e., the ability to transmit a twisting force along the length of the catheter. Sufficient torque control enables controlled maneuverability of the catheter by the application of a twisting force at the proximal end of the catheter that is transmitted along the catheter to its distal end. The need for greater torque control often conflicts with the need for reduced rigidity to prevent injury to the body vessel.

Catheters are used increasingly for medical procedures involving the human heart. In these procedures a catheter is typically advanced to the heart through veins or arteries and then is positioned at a specified location within the heart. Typically, the catheter is inserted in an artery or vein in the leg, neck, upper chest or arm of the patient and threaded, often with the aid of a guidewire or introducer, through various arteries or veins until the tip of the catheter reaches the desired location in the heart.

The distal end of a catheter used in such a procedure is sometimes preformed into a desired curvature so that by torquing the catheter about its longitudinal axis, the catheter can be manipulated to the desired location within the heart or in the arteries or veins associated with the heart. For example, U.S. Patent No. 4,882,777 discloses a catheter with a complex curvature at its distal end for use in a specific procedure in the right ventricle of a human heart. U.S. Patent No. 5,231,994 discloses a guide catheter for guiding a balloon catheter for the dilation of coronary arteries. U.S. Patent No. 4,117,836 discloses a catheter for the selective coronary angiography of the left coronary artery and U.S. Patent Nos. 5,215,540, 5,016,640 and 4,883,058 disclose catheters for selective coronary angiography of the right coronary artery. U.S. patent No. 5,242,441 discloses a deflectable catheter for ablation procedures in the ventricular chamber. See also U.S. Patent No. 4033331. In addition, U.S. Patent No. 4,898,591 discloses a catheter with inner and outer layers containing braided portions. The '591 patent also discloses a number of different curvatures for intravascular catheters. Thus, there are a number of patents which disclose catheters with predetermined shapes, designed for use during specific medical procedures generally associated with the heart or the vascular system. Because of precise physiology of the heart and the vascular system, catheters or introducers with precisely designed shapes for predetermined uses within the human heart and vascular system are increasingly important.

Catheter ablation of human type 1 atrial flutter has been disclosed in Feld, Gregory K. Radiofrequency Catheter Ablation of Cardiac Arrhythmias, Chapter 25, pages 459-476 (1995). It is believed that type 1 atrial flutter results from a counterclockwise reentry circuit in the right atrium which travels between the inferior vena cava and the tricuspid valve. Dr. Feld proposes the elimination of type 1 atrial flutter by ablation of sites posterior or inferior to the ostium of the coronary sinus between the inferior vena cava and the tricuspid valve using a catheter but without using a shaped sheath or guiding introducer. See also Feld, Gregory K. et al., "Radiofrequency Catheter Ablation for the Treatment of Human Type 1 Atrial Flutter" Circulation, Vol. 86, #4, pp. 1223-1240 (Oct. 1992) and Saoudi, N. et al., "Catheter Ablation of the Atrial Myocardium in Human Type I Atrial Flutter" Circulation, Vol. 81, #3, pp. 762-771 (March 1990).

Catheter ablation of accessory pathways using a long vascular sheath by means of a transseptal or retrograde approach is discussed in Saul, J.P., et al. "Catheter Ablation of Accessory Atrioventricular Pathways in Young Patients: Use of long vascular sheaths, the transseptal approach and a retrograde left posterior parallel approach" J. Amer. Coll. Card., Vol. 21, no. 3, pps 571-583 (March 1, 1993). See also Swartz, J.F. "Radiofrequency Endocardial Catheter Ablation of Accessory Atrioventricular Pathway Atrial Insertion Sites" Circulation, Vol. 87, no. 2, pps. 487-499 (February, 1993).

Atrial fibrillation is the most common sustained heart arrhythmia. It is estimated to occur in upwards of 0.4 percent of the adult population and perhaps as many as 10 percent of the population who are 60 years or older. Cox, J.L., et al., Electrophysiology, Pacing and Arrhythmia, "Operations for Atrial Fibrillation," Clin. Cardiol. 14, 827-834 (1991). Atrial arrhythmia may be transient or persistent. While most atrial arrhythmia occurs in individuals having other forms of underlying heart disease, some atrial arrhythmias do not directly cause death as frequently as ventricular arrhythmias. They increase the risk factor for a number of other diseases such as strokes, thrombosis, atherosclerosis, systemic and cerebral embolism and cause a number of additional medical problems.

Certain patients with symptomatic or life threatening atrial arrhythmias cannot be adequately treated by drugs or these medical devices. Other forms of aggressive treatment are mandated, which may include surgery. For example, a surgical procedure for the treatment of atrial arrhythmia known as the "Maze" procedure is disclosed in Cox, J.L. et al., Electrophysiology, Pacing and Arrhythmia, "Operations for Atrial Fibrillation," Clin. Cardiol. 14, 827-834 (1991). Other surgical procedures for atrial arrhythmia are disclosed, for example, in Martin, D., et al., Atrial Fibrillation, pp. 54-56 (1994).

Another procedure used for certain types of cardiac arrhythmia (but not atrial fibrillation) within the last 10 to 15 years is catheter ablation. This procedure has been used to interrupt or modify existing conduction pathways associated with ventricular arrhythmias within the heart. The particular area for ablation depends on the type of underlying ventricular arrhythmia. The ablation of sites in the right atrium by use of an ablation catheter have been disclosed, as previously discussed, in Feld, Gregory K. et al., "Radiofrequency Catheter Ablation for the Treatment of Human Type 1 Atrial Flutter" Circulation, Vol. 86, #4, pp. 1223-1240 (Oct. 1992) and Saoudi, N. et al., "Catheter Ablation of the Atrial Myocardium in Human Type I Atrial Flutter" Circulation, Vol. 81, #3, pp. 762-771 (March 1990). In addition, one common ablation procedure is for the treatment of atrioventricular (AV) nodal reentrant tachycardia. With this problem ablation of the fast or slow AV nodal pathways has become an accepted treatment. See Singer, I., et al., "Catheter Ablation for Arrhythmias" Clinical Manual of Electrophysiology, pp. 421-431 (1993). The use of ablation catheters for ablating locations within the heart has been disclosed, for example in U.S. Patent Nos. 4,641,649, 5,263,493, 5,231,995, 5,228,442 and 5,281,217. However, none utilize a guiding introducer to guide the ablation catheter to a particular location.

U.S. Patent No. 4,641,649 discloses the use of high frequency energy for the treatment of tachycardia or cardiac dysrhythmia. See also U.S. Patent Nos. 5,246,438 and 4,945,912 for the use of radiofrequency energy for ablation of cardiac tissue. In addition, various article's have disclosed the ablation of specific locations within the heart by use of energy, in particular, radiofrequency energy. See, for example, Gallagher, J.J. et al. "Catheter Technique for Closed-Chest Ablation of - the Atrioventricular Conduction System" N. Engl. J. Med. Vol. 306, pp. 194-200 (1982); Horowitz, L.N. "Current Management of Arrhythmia" pp. 373-378 (1991); Falk, R.H. et al. "Atrial Fibrillation Mechanics and Management" pp. 359-374 (1992); and Singer, I. "Clinical Manual of Electrophysiology" pp. 421-431 (1993).

In addition, the use of radiofrequency ablation energy for the treatment of Wolff-Parkinson-White Syndrome in the left atrium by use of a transseptal sheath is disclosed in Swartz, J.F. et al. "Radiofrequency Endocardial Catheter Ablation of Accessory Atrioventricular Pathway Atrial Insertion Sites" Circulation 87:487-499 (1993). See also Tracey, C.N. "Radio Frequency Catheter Ablation of Ectopic Atrial Tachycardia Using Paced Activation Sequence Mapping" J. Am. Coll. Cardiol. 21:910-917 (1993).

Accordingly, it is an object of this invention to prepare a guiding introducer for selected medical procedures in the right atrium.

It is a further object of this invention to prepare a guiding introducer for use in selected electrophysiology procedures within the right atrium of the heart.

Another object of this invention is to prepare a guiding introducer for use in selected ablation procedures within the right atrium of the heart.

It is a still further object of this invention to prepare a guiding introducer for use in the selected ablation of sites in the right atrium of the heart for the treatment of atrial flutter.

It is a still further object of this invention to prepare a guiding introducer for use in the right atrium as an element of the treatment of atrial arrhythmia including, specifically, atrial fibrillation.

These and other objects are obtained by the design of the guiding introducer system disclosed in the instant invention.

### Summary of Invention

The instant invention includes a device for the diagnosis and treatment of atrial flutter or atrial fibrillation within the right atrium of the heart. A method wherein such a device can be used comprises :
(a) introducing into the right atrium a guiding introducer, wherein said guiding introducer (10) contains a lumen running lengthwise therethrough, a proximal and a distal end;
(b) introducing into the lumen of the guiding introducer an ablating or mapping catheter containing a proximal and distal end, wherein said catheter has one or more electrodes located at or near the distal end of the catheter;
(c) guiding the catheter to a selected location within the right atrium by use of the guiding introducer; and
(d) mapping and/or ablating a selected location or track within the right atrium by use of the electrodes of the ablation catheter.

The instant invention also discloses a specifically designed shape or shapes for the guiding introducer for use with mapping and/or ablation catheters in the mapping and/or treatment of atrial flutter or atrial fibrillation.

### Brief Description of the Drawings

Figure 1A is a cut away view of the heart with a portion of the inferior vena cava also cut away showing the guiding introducer 10 of the instant invention supporting a catheter for an ablation and/or mapping procedure in the right atrium.

Figure 1B is a cut away view of the heart with a portion of the inferior vena cava also cut away showing the guiding introducer 10 of the instant invention supporting a catheter 12 for an ablation and/or mapping procedure in the right atrium between the tricuspid valve and the inferior vena cava and down into the inferior vena cava.

Figure 1C is a side view of the guiding introducer 10 of the instant invention within the inferior vena cava.

Figure 1D is a side view of a comparison guiding introducer 14 disclosed in Figures 5A and 5B of copending Serial No. 08/272,019. Now US Patent No. 5,575,766.

Figure 2A is a side view of the guiding introducer 10 for use in the treatment of atrial flutter or atrial fibrillation in the right atrium, wherein the side port tubing attached to the proximal end of the guiding introducer 10 is located directly behind the first section 20 of the guiding introducer 10.

Figure 2B is a side view of the guiding introducer 10 of Figure 2A rotated 90° clockwise from the position of Figure 2A, when viewed from the perspective of the proximal end 16 of the guiding, introducer 10, such that the side port is directed to the left of the guiding introducer.

Figure 2C is an end view of the guiding introducer 10 viewed from the top of the guiding introducer such that the side port tubing is directed to the left of the guiding introducer.

### Description of the Invention.

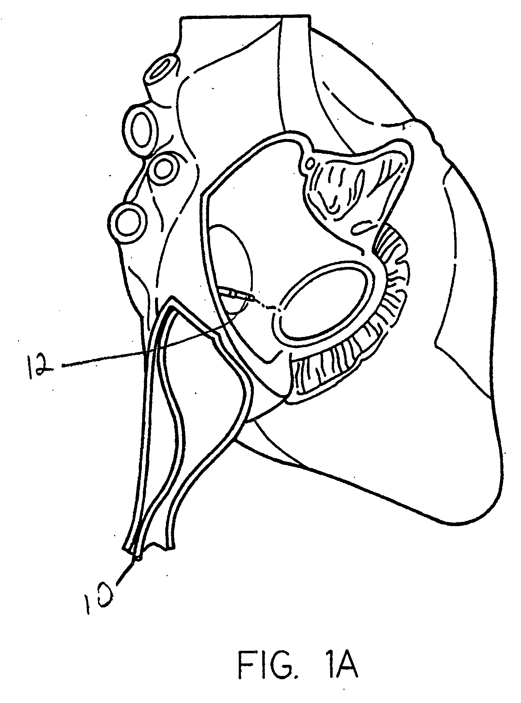

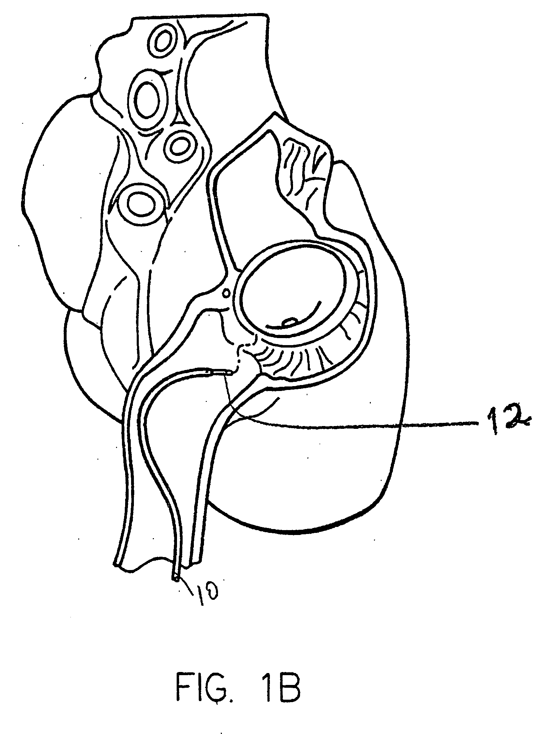

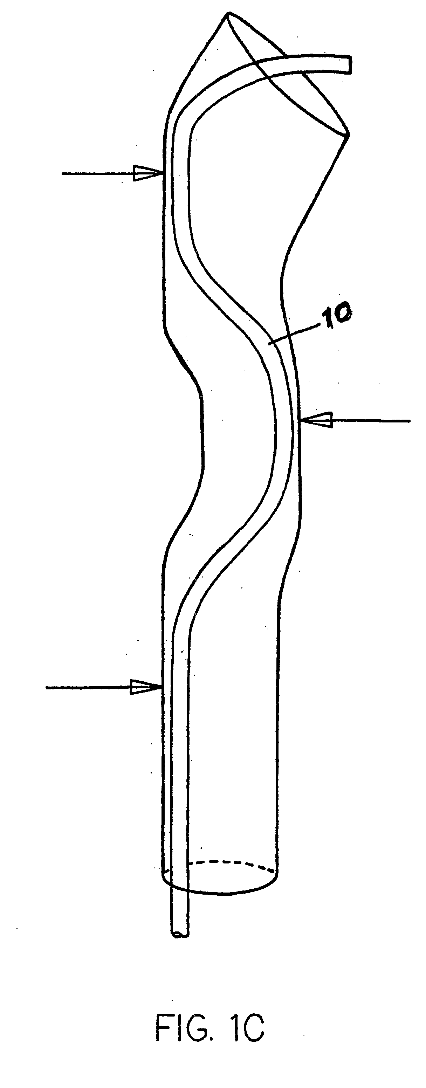

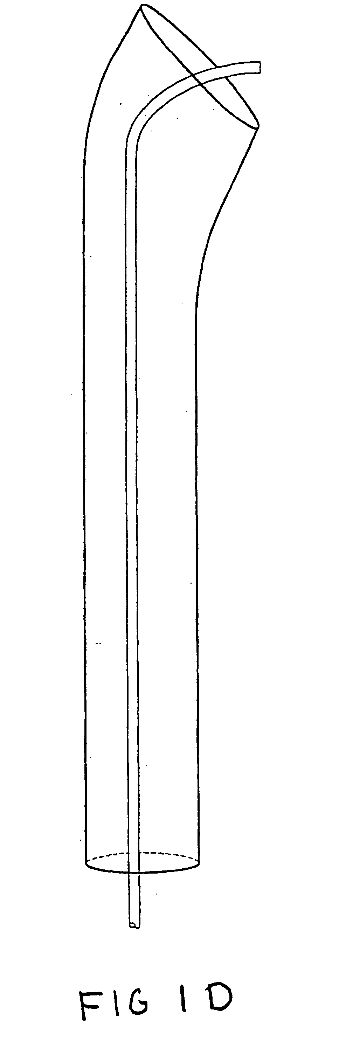

A typical human heart includes a right ventricle, a right atrium, left ventricle and left atrium. The right atrium is in fluid communication with the superior vena cava and the inferior vena cava. The atrioventricular septum separates the atria from the ventricles. The tricuspid valve contained within the atrioventricular septum communicates the right atrium with the right ventricle. The mitral valve contained within the atrioventricular septum communicates the left atrium with the left ventricle. On the inner wall of the right atrium, where it is connected with the left atrium, is a recessed portion, the fossa ovalis. Between the fossa ovalis and the tricuspid valve is the opening or ostium for the coronary sinus. The coronary sinus is a large epicardial vein which accommodates most of the venous blood which drains from the heart itself into the right atrium. See Figures 1A and 1B.

In the normal heart, contraction and relaxation of the heart muscle (myocardium) takes place in an organized fashion as electrochemical signals pass sequentially through the myocardium from the atrial to the ventricular tissue along a well defined route which includes the His-Purkinje system. Initial 'electric impulses are generated at the sinuatrial (SA) node and conducted to the atrioventricular (AV) node. The AV node lies near the ostium of the coronary sinus in the interatrial septum in the right atrium. The His-Purkinje system begins at the AV node and follows along the membranous interatrial septum toward the tricuspid valve through the atrioventricular septum and into the membranous interventricular septum. At about the middle of the ( interventricular septum, the His-Purkinje system splits into right and left branches which straddle the summit of the muscular part of the interventricular septum.

Sometimes abnormal rhythms occur in the heart which are referred to as arrhythmia. For example, patients diagnosed with Wolff-Parkinson-White syndrome have an arrhythmia, the cause of which is believed to be the existence of an anomalous conduction pathway or pathways that connect the atrial muscle tissue directly to the ventricular muscle tissue, thus by-passing the normal His-Purkinje system. These pathways are usually located in the fibrous tissue that connect the atrium and the ventricle.

Three of the most common arrhythmia are ectopic atrial tachycardia, atrial fibrillation and atrial flutter. Atrial fibrillation can result in significant patient discomfort and even death because of a number of associated problems, including: (1) an irregular heart rate which causes the patient discomfort and anxiety, (2) loss of synchronous atrioventricular contractions which interferes with cardiac hemodynamics, resulting in varying levels of congestive heart failure, and (3) stasis of blood flow, which increases the vulnerability to thromboembolism. It is sometimes difficult to isolate a specific pathological cause for the atrial fibrillation although it is believed that the principle mechanism is one or a multitude of reentry circuits within the left and/or right atrium. Efforts to alleviate these problems in the past have included significant usage of pharmacological treatments. While pharmacological treatments are sometimes effective, in some circumstances drug therapy is ineffective and frequently is plagued with side effects such as dizziness, nausea, vision problems and other difficulties.

Another type of atrial arrhythmia is atrial flutter. Atrial flutter is thought to result from a counterclockwise reentry circuit in the right atrium associated with the atrial septum and the right atrial freewall. The reentry circuit normally travels between the inferior vena cava and the tricuspid valve. Studies have demonstrated that the reentry circuit contains an'area of slow conduction generally in the low posteroseptal right atrium. Endocardial direct current energy has been used successfully in the treatment of atrial flutter because it produces a large, local area of ablation and generally converts the atrial flutter to sinus rhythm. (See the Saoudi, et al. article.) Dr. Feld in his articles proposes the use of radio frequency energy for the treatment of atrial flutter. In particular, Dr. Feld suggests ablation in two particular sites, posterior or inferior to the ostium of the coronary sinus between the inferior vena cava and the tricuspid valve.

In the last few years surgical procedures have also been utilized in the treatment of atrial arrhythmia. The goal of these surgical procedures parallel that of the pharmacological treatments, 'to relieve both the subjective symptoms of atrial arrhythmia as well as to normalize hemodynamics by restoring regular atrial contributions to the cardiac output. One method suggested requires isolation of the left atrium from the remainder of the heart by a surgical procedure. See Cox, J.L., et al., "The Surgical Treatment of Atrial Fibrillation," J. Thoracic and Cardiovascular Surgery, Vol. 101, No. 4, p. 570 (1991). The initial incisions followed by the scar tissue left by such surgery effectively isolates the left atrium and, in some cases, provides some relief for the patient. Such relief can occur as long as the right atrium maintains adequate sinus rhythm. Various problems associated with this procedure, other than the maintenance of appropriate sinus rhythm, include thromboembolic risks.

Another procedure for treatment of atrial arrhythmia involves the ablating of the His bundle. A permanent pacemaker is then installed, resulting in a regular ventricular beat. See Cox, J.J., et al., "The Surgical Treatment of Atrial Fibrillation," Journal of Thoracic and Cardiovascular Surgery, Vol. 101, No. 4, pp. 570-572 (1991). However, because the atria may continue to fibrillate, normal cardiac hemodynamics is not restored and there is still vulnerability to thromboembolism.

A newer surgical procedure for the treatment of atrial arrhythmia designed by Guiraudon in 1985 results in the creation of a narrow corridor between the SA node and the AV node. See Guiraudon, G.M., et al., Combined Sinoatrial Node/Atrial Ventricular Node Isolation: a Surgical Alternative to His Bundle Ablation in Patients with Atrial Fibrillation; Circulation 72:(pt-2) III-220 (1985). This procedure isolates a narrow corridor from the remainder of the atrial muscle tissue and can, in some circumstances, alleviate some of the problems associated with atrial arrhythmia.

A more recent, more complex surgical procedure, the "Maze" procedure, has also been designed to treat atrial arrhythmia, particularly atrial fibrillation. See Cox, J.L., et al., "The Surgical Treatment of Atrial Fibrillation," Journal of Thoracic and Cardiovascular Surgery, Vol 101, pp. 569-83 (1989). Appropriately placed atrial incisions in the myocardium are designed to interrupt the conduction routes of those areas in the atria that produce the most common reentrant circuits. The procedure is also designed to direct the sinus impulse from the sinus node to the AV node along a specified route. After the procedure, the entire atrial myocardium (except for the atrial appendages and pulmonary veins) is designed to be electrically active by providing for multiple blind alleys off the main conduction route between the SA node and the AV node, thereby preserving atrial transport function postoperatively. While this procedure has resulted in successful treatments for certain patients, there are significant potential risks due to the extensive nature of the surgery.

The effectiveness of the "Maze" procedure is dependent upon the destruction of tissue within the atrium along specific lines or tracks to prevent the formation of reentry circuits while still allowing the atria to contract and permitting the return of normal atrio-ventricular conductivity. It has been discovered that similar success can be achieved without invasive surgery by the use of ablation procedures performed within the atria. However, to accomplish this procedure the ablation catheter must be positioned at pre-determined locations within the atria to ablate a predetermined location or tracks within the atria, thus forming a natural barrier to the formation of the reentry circuits. In addition to the necessity of producing ablation tracks in well defined areas of atria, it is also critical for proper transmural lesion formation that adequate contact pressure be maintained between the ablation catheter electrode and the heart tissue to be ablated.

The ablation catheters used to perform the ablation procedures produce scar tissue at the selected site within the atria. The energy necessary to scar or ablate the tissue can be provided by a number of different sources. Originally direct current was utilized to provide the energy for ablation procedures. More recently the preferred choice of energy source has been radio frequency energy (R.F.). Laser, microwave, ultrasound and direct current energy procedures including low energy direct, high energy direct and fulgutronization procedures have also been utilized or are being considered for ablation procedures. The preferred source of energy for the ablation procedures of the instant invention is RF energy.

Individual applications of ablation energy are frequently unsuccessful at blocking the reentrant circuit, and the ablation procedures may have to be' repeated several times. This may be because the catheter electrode is not positioned correctly at the beginning of the energy application or that the catheter electrode moves during the attempted ablation. Another problem can be that even though the catheter electrode is located correctly, there may not be enough contact pressure between the tissue to be ablated and the electrode to facilitate sufficient current to flow through the tissue to properly ablate said tissue. The procedure may also be very time consuming. In addition, it may require catheter exchanges to replace a defective or inappropriately selected catheter. To effectively ablate the atrial tissue, the ablation catheter must be positioned precisely within the atrium and maintained in contact with the atrial tissue throughout the energy application. Such procedures may require the ablation electrode of the ablation catheter to remain in contact with the atrial tissue for an extended period of time.

Currently, the most common approach for the positioning of an ablation catheter in the right atrium calls for the introduction of the medical device into the right femoral vein and advancement up through the inferior vena cava into the right atrium. Although a superior approach to the right atrium can also be used, for purposes of the guiding introducer of the instant invention, the inferior approach through the inferior vena cava is required.

Mere introduction of the ablation and mapping catheter into the right atrium is not sufficient to effectively and efficiently perform the ablation procedures on the reentry circuits. The medical practitioner commonly monitors the introduction of the catheter and its progress through the vascular system by a fluoroscope. Such fluoroscopes can not easily identify the specific features of the heart in general, and the critically important structures of the right atrium in specific, thus making placement of the ablation electrode difficult. This placement is especially difficult as the beating heart is in motion. In addition, the catheter will be moving within the right atrium as blood is being pumped through the heart throughout the procedure. Further, because of the difficulty of properly locating the catheter, extended exposure to fluoroscopy may be inevitable. However, such excessive exposure is certainly undesirable.

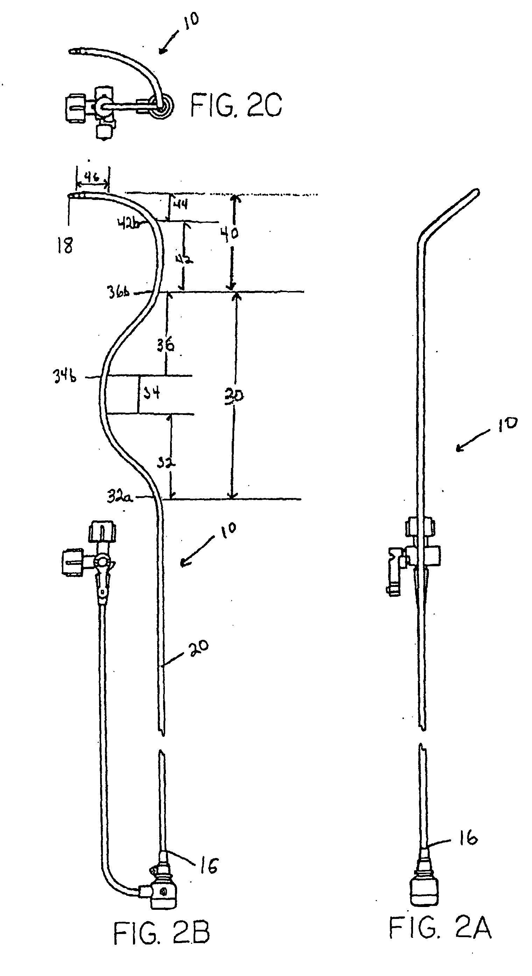

The guiding introducer 10 system of the instant invention addresses and solves these problems. Referring now to Figures 2A, 2B and 2C, the guiding introducer 10 of the present invention for use in the right atrium for the treatment of atrial flutter (and also for the treatment of atrial fibrillation) is comprised of a first 20, second 30 and third sections 40. (Each section is preferably formed as an integral portion of the entire guiding introducer without discrete divisions. However, the division of the guiding introducer into different sections for discussion better illustrates the overall shape of the guiding introducer.) The guiding introducer 10 will be shown in three views. In each of the views for ease of analysis, the guiding introducer 10 will be secured to a valve for attachment to a conventional side port tubing and stop cock. In each such arrangement, the shape of the guiding introducer 10 and each of its sections will be described, making reference to its position in relation to the side port and side port tubing, where the proximal end 16 of the guiding introducer 10 is secured to the side port tubing. In the first referenced figure (Figure 2A), the side port tubing is viewed as if it is behind the first section 20 of the guiding introducer 10. In the second figure (Figure 2B) the guiding Introducer 10 is rotated clockwise about the axis of the first section 20 of the guiding introducer when viewed from the perspective of the proximal end of the guiding introducer. In the third figure (Figure 2C) the guiding introducer is rotated upwards around the distal end of the guiding introducer.

The first section 20 of the guiding introducer is a conventional, elongated, hollow, generally straight section of sufficient length for introduction into the patient and for manipulation from the point of insertion to the specific desired location within the heart. (The overall length of the first section 20 as shown in Figures 2A and 2B has been reduced for ease of illustration.)

Merged with the distal end of the first section 20 of the guiding introducer 10 is the second section 30 which is a generally C-shaped section, curving to the left and then back to the right to a point where it meets with what would have been the first section 20, if that first section 20 had been extended. See Figure 2B. The purpose of this second section is to rest against one side of the inferior vena cave at the same time as a portion of the first and third sections rest against the opposite side of the inferior vena cava. Its overall shape is not particularly critical. However, in a preferred embodiment, this second section is comprised of three portions. The first portion curves first to the left in a curved segment as shown in Figure 2B in a radius from 1.9 to 4.5 cm (0.75 to 1.75 in.) and preferably from 2.5 to 3.8 cm (1.00 to 1.50 in.). The extent of the arc of this curved segment is from 30 to 90 degrees and preferably from 30 to 60 degrees. Following the curving of this curved segment to the left, the second section then curves back to the right in a second curved segment with a radius from 1.9 to 4.5 cm (0.75 to 1.75 in.) and preferably from 2.5 to 3.8 cm (1.00 to 1.5 in.). The extent of the arc of this second curved segment is from 30 to 90 degrees and preferably from 30 to 60 degrees of arc. In a preferred embodiment, both the first and the second curved segments of this first portion are generally coplanar (within about 15 degrees of coplanar). At the distal end of this first portion of the second section begins the second portion which is a straight section from 0.2 to 5.1 cm (0.1 to 2.0 in.) and preferably from 0.2 to 1.3 cm (0.1 to 0.5 in.). The overall length of this second section is not critical and it may, in fact, be eliminated, if desired. If the first section of the guiding introducer were extended, this second straight portion would be 1.2 to 5.1 cm (0.5 to 2.0.), and preferably from 1.2 to 3.8 cm (0.5 to 1.5 in.), away from the first section extended. Preferably, this straight second portion is also coplanar with the first portion. At the distal end of this second portion begins the third portion of the second section. This third portion is preferably a mirror image of the first portion, curving first to the right and then back to the left as shown in Figure 2B. The radii and the curvature of the arc of this third portion are approximately the same as those of the first portion of the second section only in reverse.

At the distal end of this second portion, the guiding introducer returns generally to a position that would correspond with the first section of the guiding introducer if that first section were extended.

The distance from the proximal end of the first portion of the second section to the distal end of the third portion of the second section is 5.0 to 17.8 cm (2.0 to 7.0 in.) and preferably 8.9 to 12.7 cm (3.5 to 5.0 in.) in length. The overall length of this second section is not particularly critical. While in a preferred embodiment the first, second and third portions of the second section are coplanar (within about 15 degrees of coplanar), alternatively, the first, second and third sections may be out-of-plane with each other. The overall shape of this second section is not particularly critical as long as a portion of the second section is 1.2 to 5.1 cm (0.5 to 2.0 in.), and preferably from 1.2 to 3.8 cm (0.5 to 1.5 in.), away from an extension of the first section if extended. The purpose of this second section is to fit against one side of inferior vena cava while a portion of the first section and the third section of the guiding introducer rests against the opposite side of the inferior vena cava, thereby providing support for the guiding introducer while it is in the inferior vena cava. Although the second section is preferably a pair of curved portions surrounding a short straight portion, alternatively three or more separate curved portions, each with the same or different curvatures and radii, can be used alone or in combination with one or more straight portions, or no straight portion at all, as long as the combination of separate curves and straight portions creates a device that rests against both sides of the inferior vena cava.

The first and second sections are preferably coplanar (within about 15 degrees of coplanar).

The third section of this guiding introducer is also divided into three portions. Preferably, the first portion begins with a straight segment from 0.2 to 9.6 cm (0.1 to 3.0 in.) and preferably from 0.2 to 1.3 cm (0.1 to 0.5 in.) in length. Depending on the length of the straight portion of the second section and the overall length of the second section, the length of this first straight portion can vary dramatically. In fact, no first straight portion need exist at all. The first portion then curves to the right, preferably out of the plane of the first and second sections as shown in Figure 2A with a radius from 0.5 to 2.5 cm (0.2 to 1.0 in.) and preferably from 0.7 to 1.8 cm (0.3 to 0.7 in.). The extent of the arc of this curve is from 20 to 90 degrees and preferably from 40 to 60 degrees. The distal end of this first portion of the third section begins the second portion of the third section 40 wherein the guiding introducer 10 curves to the left as shown in Figures 2B and 2C. The curve of this second portion 44 has a radius from about 1,2 to about 3,8 cm (0.5 to about 1.5 in.) and preferably from about 2.0 to about 3.1cm (0.8 to about 1.2 in.) with an arc of the curve from about 45 to about 135 degrees, and preferably from about 70 to about 110 degrees of arc, ending in the third portion 46 of the third section 40. The third portion 46 of the third section is a straight section from about 0.2 to about 5.1 cm (0.1 to about 2.0 in.) in length and preferably, from about 0.5 to about 2.6 cm (0.2 to about 1.0 in.), ending in the distal tip of the guiding introducer. As.with other straight portions of the guiding introducer 10, the overall length of this straight, third portion 46 can vary dramatically or can even be eliminated, if desired. As with the second section 30 of the guiding introducer 10, a plurality of curves and straight portions can replace the curved portions and straight portion of the preferred embodiment of the third section 40 of the guiding introducer 10 as long as the combination of curves and straight sections creates generally the same overall curvature of the third section.

The distal tip 18 of the guiding introducer 10 may be, and preferably will be, tapered to form a good transition with a dilator. This tapering is preferably less than 10° and more preferably about 4° to about 7°. The guiding introducer 10 preferably also contains one or a plurality of radiopaque tip marker bands near the distal tip of the guiding introducer. This guiding introducer 10 also preferably contains one or a plurality of vents near the distal tip 18 of the guiding introducer 10, preferably three or four such vents. The vents are preferably located no more than about 2.5 cm (1.00 in.) from the distal tip of the guiding introducer and more preferably 0.2 to about 2.5 cm (0.10 to about 1.00 in) from the distal tip. The size of these vents should be in the range of about 0.1 to about 0.15 cm (40 to about 60/1000 of an inch) in diameter. These vents are designed to prevent air from entering the guiding introducer 10 caused by the withdrawal of the catheter 12 contained within the guiding introducer 10 in the event the distal end 18 of the guiding introducer 10 is occluded. For example, if the tip 18 of the guiding introducer is placed against the myocardium and the catheter 12 located within the guiding introducer 10 is withdrawn, a vacuum may be created within the guiding introducer 10 if no vents are provided. If such vacuum is formed, air may be forced back into the guiding introducer 10 by the reintroduction of a catheter 12 into the lumen of the guiding introducer 10. Such air could cause significant problems in the patient, including the possibility of a stroke, heart attack or other such problems common with air embolisms. The addition of vents near the distal tip 18 of the guiding introducer 10 prevents the formation of such vacuum by permitting fluid, presumably blood, to be drawn into the lumen of the guiding introducer as the catheter 12 is being removed from the guiding introducer 10, thus preventing the creation of a vacuum which could cause air to enter the guiding introducer.

The guiding introducer 10 may be made of any material suitable for use in humans which has a memory or permits distortion from, and substantial return to, the desired three dimensional shape. For the purpose of illustration and not limitation, the internal diameter of the guiding introducer may vary from about 6 to about 12 "French" respectively (1 French equals 1/3 of a millimeter). Such guiding introducer 10 can also accept dilators and appropriate guidewires. Obviously, if larger or smaller dilators or catheters are used in conjunction with the guiding introducer 10 of the instant invention, modifications in size or shape can be made to the guiding introducer.

Variations in size and shape of the guiding introducer 10 are also intended to encompass pediatric uses, although the preferred uses are for adult human hearts. It is well recognized that pediatric uses may require reductions in size of the various sections of the guiding introducer 10, in particular the first section, but without significant modifications to the shape or curve of the guiding introducer.

In addition, variations in size or shape of the guiding introducer 10 are also intended to encompass the specialized situations that sometimes occur in patients with enlarged or rotated hearts.

In operation, a modified Seldinger technique is normally used for the insertion of the catheter into the right femoral vein. The appropriate vessel is accessed by needle puncture. A soft flexible tip of an appropriately sized guidewire is then inserted through, and a short distance beyond, the needle into the vessel. Firmly holding the guidewire in place, the needle is removed. The guidewire is then advanced through the vein up to the inferior vena cava and into the right atrium. With the guidewire in place, a dilator is then placed over the guidewire with the guiding introducer placed over the dilator. The dilator and guiding introducer 10 generally form an assembly to be advanced together along the guidewire into the right atrium. After insertion of the guiding introducer 10, the guidewire and dilator are then withdrawn. The catheter 12 to be used for treatment of atrial flutter or atrial fibrillation is advanced through the lumen of the guiding introducer 10 and is placed at an appropriate location in the right atrium.

By movement of the guiding introducer 10 in conjunction with fluoroscopic viewing, the distal portion of the guiding introducer 10 can be manipulated to direct the distal end of a catheter 12 placed within the lumen of the guiding introducer 10 to a specific internal surface within the right atrium. In addition, by providing sufficient rigidity, the distal end of the guiding introducer 16 can be maintained in that fixed location or surface position of the endocardial structure to permit the appropriate procedures to be performed. If sensing procedures are involved, the guiding introducer 10 is maneuvered to the desired location. At that point, the electrical activity of the heart peculiar to that location can be precisely determined by use of an electrophysiology catheter placed within the guiding introducer. Further, as the guiding introducer 10 permits precise location of catheters, an ablation catheter may be placed at a precise location for destruction of the cardiac tissue by the use of energy, for example, radio frequency, thermal, laser or direct current (high energy direct, low energy direct and fulgutronization procedures). Preferably, radio frequency energy is utilized for the ablation procedure.

The ablation procedures for ablation of atrial flutter are best shown in Figures 1A and 1B. In each, the guiding introducer 10 of the instant invention directs the ablation catheter 12 to the lip of the inferior aspect of the tricuspid valve, across the isthmus of tissue between the tricuspid valve and the inferior vena cava and down into the inferior vena cava past the transition point between contractile myocardium and the vascular wall of the inferior vena cava. By this procedure a block in a counterclockwise reentry circuit in the right atrium is formed, thus eliminating the reentry circuit which is thought to cause atrial flutter.

The precise placement of the ablation catheter 12 electrode is important as there will be no dilution of the energy delivered due to unfocused energy being dissipated over the entire cardiac chamber and lost in the circulating blood by a constantly moving tip of the ablating catheter 12. This guiding introducer 10 permits a significantly reduced amount of energy to be applied while still achieving efficient ablation. Further, time used to perform the procedure is significantly reduced over procedures where no guiding introducer is used. This reduction in time also reduces the amount of fluoroscopy that is necessary for the procedure. The precise placement of the ablation catheter 12 within the right atrium is particularly important because of the difficulties associated with the ablation of atrial flutter.

In addition to the use of the guiding introducer 10 of the instant invention for the treatment of atrial flutter, this guiding introducer may also be used in the treatment of atrial fibrillation. In copending application Serial No. 08/272,014 Now US Patent No 5,575,766, a procedure for the treatment of atrial fibrillation by using guiding introducers and ablation catheters is disclosed. In that procedure a number of different ablation tracks are formed in the left and right atria, each of which is designed to eliminate specific reentry circuits which are thought to cause atrial fibrillation. One of the ablation procedures that is performed as an element of the treatment of atrial fibrillation in the copending application is the ablation of a track between the tricuspid valve and the inferior vena cava. This track, as shown in the copending application in Figure 3E'is referred to as "track five." The guiding introducer of the instant invention is an alternative for the guiding introducer disclosed in the copending application in Figures 5A and 5B. In particular, the addition to the guiding introducer of the copending application of the generally C-shaped bend, referred to as the second section of the instant guiding introducer, assists in holding the guiding introducer of the instant invention in a fixed location within the inferior vena cava. (In contrast, the guiding introducer of the copending application without the generally C-shaped bend is shown in Figure 1D.) As is shown in Figure 1C, this bend, comprising the second section of the guiding introducer acts to hold the guiding introducer in place against one side of the inferior vena cava while a portion of the first section and the third section of the guiding introducer of the instant invention are held against the opposite side of the inferior vena cava, thus providing a secure platform for an ablation catheter to create the appropriate ablation track across the isthmus between the tricuspid valve and the inferior vena cava.

It will be apparent from the foregoing that while particular forms of the invention have been illustrated and described, various modifications can be made without departing from the scope of the invention. Accordingly, it is not intended that this invention be limited except as by the appended claims.

## Claims

1. A guiding introducer (**10**) for use with an ablating or mapping catheter for the diagnosis and/or treatment of atrial flutter or atrial fibrillation in the right atrium comprising a first, (**20**) second (**30**) and third (**40**) sections, wherein the second section (**30**) of the guiding introducer comprises a first, (**32**) second (**34**) and third (**36**) portion, wherein the first portion (**32**) of the second section (30) comprises a first curved segment followed by a second curved segment curving generally in the opposite direction from the first curved segment, and wherein the third portion (**36**) of the second section (**30**) comprises a first curved segment and a second curved segment curving generally in the opposite direction from the first curved segment of the third portion (**36**).

2. The guiding introducer (**10**) of Claim 1 wherein the first section (**20**) of the guiding introducer is an elongated, hollow, generally straight section of sufficient length for introduction into the patient and for manipulation from the point of insertion through to a desired location within the heart.

3. The guiding introducer (**10**) of Claims 1 or 2 wherein the first curved segment of the first portion (**32**) of the second section (**30**) curves with an overall arc of 30 to 90 degrees, wherein the second curved segment curves with an overall arc of 30 to 90 degrees, wherein the second portion (**34**) of the second section (**30**) comprises a straight portion from 0.2 to 5.1 cm. (0.1 to 2.0 inches) in length, wherein the first curved segment of the third portion (**36**) of the second section (**30**) curves in an overall arc of 30 to 90 degrees, and wherein the second curved segment curves with an overall arc of 30 to 90 degrees.

4. The guiding introducer (10) of any one of Claims 1 to 3 wherein the first (**20**) and second (**30**) sections are substantially coplanar and/or wherein the overall lineal length of the second section (**30**) is from 5.1 to 17.8 cm. (2.0 to 7.0 inches).

5. The guiding introducer (**10**) of any one of Claims 1 to 4 wherein the second section (**30**) of the guiding introducer comprises one or more straight portions and one or more curved portions.

6. The guiding introducer (**10**) of any one of Claims 1 to 5 wherein the third section (**40**) comprises a first, (**42**) second (**44**) and third (**46**) portion and wherein the first portion (**42**) of the third section (**40**) comprises a straight segment from 0.2 to 7.6 cm. (0.1 to 3.0 inches) in length, followed by a curved segment, curving in an overall arc of 20 to 90 degrees and/or wherein the first portion (**42**) of the third section (**40**) curves out of a plane formed by the first (**20**) and second (**30**) sections of the guiding introducer (**10**) 20 to 90 degrees and wherein the second portion (**44**) of the third section (**40**) is a curved portion, curving from 45 to 135 degrees.

7. The guiding introducer (10) of Claim 6 wherein the second portion (**44**) of the third section (**40**) curves out of a plane formed by the first (**20**) and second (**30**) sections of the guiding introducer (**10**) from 20 to 90 degrees and wherein the third portion (**46**) of the third section (**40**) is a generally straight portion from 0.5 to 5.1 cm. (0.2 to 2.0 inches) ending in the distal tip (**18**) of the guiding introducer (**10**).

8. The guiding introducer (10) of any one of Claims 1 to 7 wherein the third section (**40**) comprises a first (**42**) and a second (**44**) portion and wherein the first portion (**42**) of the third section (40) curves out of a plane formed by the first(**20**)and second (30) sections of the guiding introducer (**10**) 20 to 90 degrees; wherein the second portion (**44**) of the third section (**40**) is a curved portion, curving from 45 to 135 degrees and/or wherein the second portion (**44**) of the third section (**40**) curves out of a plane formed by the first (**20**) and second (**30**) sections of the guiding introducer (**10**) from 20 to 90 degrees.

9. The guiding introducer of any one of Claims 1 to 8 wherein the third portion (**46**) of the third section (**40**) is a generally straight portion from 0.5 to 5.1 cm. (0.2 to 2.0 inches) ending in the distal tip (**18**) of the guiding introducer (**10**) and/or wherein the third section (**40**) of the guiding introducer comprises one or more straight sections and one or more curved sections.

## Patentansprüche

1. (Ein-)Führungsvorrichtung (10) zur Verwendung mit einem Ablations- oder Kartierungskatheter zur Diagnose und/oder Behandlung von atriellem Flattern oder atrieller Fibrillation im rechten Atrium, enthaltend einen ersten (20), zweiten (30) und dritten (40) Abschnitt, worin der zweite Abschnitt (30) der (Ein-)Führungsvorrichtung einen ersten (32), zweiten (34) und dritten (36) Teil aufweist, worin der erste Teil (32) des zweiten Abschnitts (30) ein erstes gekrümmtes Segment enthält, gefolgt von einem zweiten gekrümmten Segment, das allgemein in der gegensätzlichen Richtung zu dem ersten Segment gekrümmt ist, und worin der dritte Teil (36) des zweiten Abschnitts (30) ein erstes gekrümmtes Segment und ein zweites gekrümmtes Segment aufweist, das im Allgemeinen in der gegensätzlichen Richtung zu dem ersten gekrümmten Segment des dritten Teils (36) gekrümmt ist.

2. (Ein-)Führungsvorrichtung (10) nach Anspruch 1, worin der erste Abschnitt (20) der (Ein-)Führungsvorrichtung ein verlängerter, hohler, im Allgemeinen gerader Abschnitt von genügender Länge zur Einführung in den Patienten und zur Handhabung ausgehend von der Stelle des Einführens bis zu der gewünschten Stelle innerhalb des Herzens ist.

3. (Ein-)Führungsvorrichtung (10) gemäß Anspruch 1 oder 2, worin das erste gekrümmte Segment des ersten Teils (32) des zweiten Abschnitts (30) mit einem Gesamtbogen von 30 bis 90 Grad gekrümmt ist, worin das zweite gekrümmte Segment mit einem Gesamtbogen von 30 bis 90 Grad gekrümmt ist, worin der zweite Teil (34) des zweiten Abschnitts (30) einen geraden Teil mit einer Länge von 0,2 bis 5,1 cm (0,1 bis 2,0 inch) aufweist, worin das erste gekrümmte Segment des dritten Teils (36) des zweiten Abschnitts (30) mit einem Gesamtbogen von 30 bis 90 Grad gekrümmt ist, und worin das zweite gekrümmte Segment mit einem Gesamtbogen von 30 bis 90 Grad gekrümmt ist.

4. (Ein-)Führungsvorrichtung (10) nach einem der Ansprüche 1 bis 3, worin der erste (20) und der zweite (30) Abschnitt im Wesentlichen coplanar sind und/oder worin die lineare Gesamtlänge des zweiten Abschnitts (30) 5,1 bis 17,8 cm (2,0 bis 7,0 inch) beträgt.

5. (Ein-)Führungsvorrichtung (10) nach einem der Ansprüche 1 bis 4, worin der zweite Abschnitt (30) der (Ein-)Führungsvorrichtung einen oder mehrere gerade Teile und einen oder mehrere gekrümmte Teile aufweist.

6. (Ein-)Führungsvorrichtung (10) nach einem der Ansprüche 1 bis 5, worin der dritte Abschnitt (40) einen ersten (42), zweiten (44) und dritten (46) Teil enthält und worin der erste Teil (42) des dritten Abschnitts (40) ein gerades Segment mit einer Länge von 0,2 bis 7,6 cm (0,1 bis 3,0 inch) enthält, gefolgt von einem gekrümmten Segment, das mit einem Gesamtbogen von 20 bis 90 Grad gekrümmt ist, und/oder worin der erste Teil (42) des dritten Abschnitts (40) aus der von dem ersten (20) und dem zweiten (30) Abschnitt der (Ein-)Führungsvorrichtung (10) gebildeten Ebene in einem Winkel von 20 bis 90 Grad heraus gekrümmt ist und worin der zweite Teil (44) des dritten Abschnitts (40) einen gekrümmten Teil mit einer Krümmung von 45 bis 135 Grad darstellt.

7. (Ein-)Führungsvorrichtung (10) nach Anspruch 6, worin der zweite Teil (44) des dritten Abschnitts (40) aus der von dem ersten (20) und dem zweiten (30) Abschnitt der (Ein-)Führungsvorrichtung (10) gebildeten Ebene in einem Winkel von 20 bis 90 Grad heraus gekrümmt ist und worin der dritte Teil (46) des dritten Abschnitts (40) einen allgemein geraden Teil mit einer Länge von 0,5 bis 5,1 cm (0,2 bis 2,0 inch) darstellt, der in der distalen Spitze (18) der (Ein-)Führungsvorrichtung (10) endet.

8. (Ein-)Führungsvorrichtung (10) nach einem der Ansprüche 1 bis 7, worin der dritte Abschnitt (40) einen ersten (42) und einen zweiten (44) Teil enthält und worin der erste Teil (42) des dritten Abschnitts (40) aus der von dem ersten (20) und dem zweiten (30) Abschnitt der (Ein-)Führungsvorrichtung (10) gebildeten Ebene in einem Winkel von 20 bis 90 Grad heraus gekrümmt ist; worin der zweite Teil (44) des dritten Abschnitts (40) einen gekrümmter Teil mit einer Krümmung von 45 bis 135 Grad darstellt, und/oder worin der zweite Teil (44) des dritten Abschnitts (40) aus der von dem ersten (20) und dem zweiten (30) Abschnitt der (Ein-)Führungsvorrichtung (10) gebildeten Ebene in einem Winkel von 20 bis 90 Grad heraus gekrümmt ist.

9. (Ein-)Führungsvorrichtung nach einem der Ansprüche 1 bis 8, worin der dritte Teil (46) des dritten Abschnitts (40) einen allgemein geraden Teil von 0,5 bis 5,1 cm (0,2 bis 2,0 inch) darstellt, der in der distalen Spitze (18) der (Ein-)Führungsvorrichtung (10) endet, und/oder worin der dritte Abschnitt (40) der (Ein-)Führungsvorrichtung einen oder mehrere gerade Abschnitte und einen oder mehrere gekrümmte Abschnitte enthält.

## Revendications

1. Dispositif d'introduction à guidage (10) pour un usage avec un cathéter d'ablation ou un cathéter de cartographie adapté pour le diagnostic et/ou le traitement'du flutter auriculaire ou de la fibrillation auriculaire dans l'oreillette droite comprenant un premier (20), un deuxième (30) et un troisième (40) tronçons, dans lequel le deuxième tronçon (30) du dispositif d'introduction à guidage comprend une première (32), une deuxième (34) et une troisième (36) parties, dans lequel la première partie (32) du deuxième tronçon (30) comprend un premier segment incurvé suivi d'un deuxième segment incurvé qui s'incurve globalement dans la direction opposée à celle du premier segment incurvé, et dans lequel la troisième partie (36) du deuxième tronçon (30) comprend un premier segment incurvé et un deuxième segment incurvé qui s'incurve globalement dans la direction opposée à celle du premier segment incurvé de la troisième partie (36).

2. Dispositif d'introduction à guidage (10) selon la revendication 1, dans lequel le premier tronçon (20) du dispositif d'introduction à guidage est une section allongée, creuse, globalement droite, qui a une longueur suffisante pour pouvoir être introduite dans le patient et pour pouvoir être manipulée depuis le point d'insertion jusqu'à un emplacement désiré à l'intérieur du coeur.

3. Dispositif d'introduction à guidage (10) selon la revendication 1 ou 2, dans lequel le premier segment incurvé de la première partie (32) du deuxième tronçon (30) s'incurve en formant un arc global de 30 à 90 degrés, dans lequel le deuxième segment incurvé s'incurve en formant un arc global de 30 à 90 degrés, dans lequel la deuxième partie (34) du deuxième tronçon (30) comprend une partie droite de 0,2 à 5, 1 cm (0,1 à 2,0 pouces) en longueur, dans lequel le premier segment incurvé de la troisième partie (36) du deuxième tronçon (30) s'incurve en formant un arc global de 30 à 90 degrés, et dans lequel le deuxième segment incurvé s'incurve en formant un arc global de 30 à 90 degrés.

4. Dispositif d'introduction à guidage (10) selon l'une quelconque des revendications 1 à 3, dans lequel le premier (20) et le deuxième (30) tronçons sont sensiblement coplanaires et/ou dans lequel la longueur linéaire globale du deuxième tronçon (30) est de 5,1 à 17,8 cm (2,0 à 7 pouces).

5. Dispositif d'introduction à guidage (10) selon l'une quelconque des revendications 1 à 4, dans lequel le deuxième tronçon (30) du dispositif d'introduction à guidage comprend une ou plusieurs parties droites et une ou plusieurs parties incurvées.

6. Dispositif d'introduction à guidage (10) selon l'une quelconque des revendications 1 à 5, dans lequel le troisième tronçon (40) comprend une première (42), une deuxième (44) et une troisième (46) parties, et dans lequel la première partie (42) du troisième tronçon (40) comprend un segment droit de 0,2 à 7,6 cm (0,1 à 3,0 pouces) en longueur, suivi d'un segment incurvé qui s'incurve en formant un arc global de 20 à 90 degrés, et/ou dans lequel la première partie (42) du troisième tronçon (40) s'incurve vers l'extérieur d'un plan formé par le premier (20) et le deuxième (30) tronçons du dispositif d'introduction à guidage (10) en formant un arc de 20 à 90 degrés, et dans lequel la deuxième partie (44) du troisième tronçon (40) est une partie incurvée qui s'incurve en formant un arc de 45 à 135 degrés.

7. Dispositif d'introduction à guidage (10) selon la revendication 6, dans lequel la deuxième partie (44) du troisième tronçon (40) s'incurve vers l'extérieur d'un plan formé par le premier (20) et le deuxième (30) tronçons du dispositif d'introduction à guidage (10) en formant un arc de 20 à 90 degrés, et dans lequel la troisième partie (46) du troisième tronçon (40) est une partie globalement droite de 0,5 à 5,1 cm (0,2 à 2,0 pouces) qui se termine dans l'embout distal (18) du dispositif d'introduction à guidage (10).

8. Dispositif d'introduction à guidage (10) selon l'une quelconque des revendications 1 à 7, dans lequel le troisième tronçon (40) comprend une première (42) et une deuxième (44) parties et dans lequel la première partie (42) du troisième tronçon (40) s'incurve vers l'extérieur d'un plan formé par le premier (20) et le deuxième (30) tronçons du dispositif d'introduction à guidage (10) en formant un arc de 20 à 90 degrés ; dans lequel la deuxième partie (44) du troisième tronçon (40) est une partie incurvée qui s'incurve en formant un arc de 45 à 135 degrés et/ou dans lequel la deuxième partie (44) du troisième tronçon (40) s'incurve vers l'extérieur d'un plan formé par le premier (20) et le deuxième (30) tronçons du dispositif d'introduction à guidage (10) en formant un arc de 20 à 90 degrés.

9. Dispositif d'introduction à guidage (10) selon l'une quelconque des revendications 1 à 8, dans lequel la troisième partie (46) du troisième tronçon (40) est une partie globalement droite de 0,5 à 5,1 cm (0,2 à 2,0 pouces) qui se termine dans l'embout distal (18) du dispositif d'introduction à guidage (10) et/ou dans lequel le troisième tronçon (40) du dispositif d'introduction à guidage comprend une ou plusieurs sections droites et une ou plusieurs sections incurvées.
